# DEMANDE DE BREVET EUROPEEN

(11) **EP 4 600 322 A1**
(43) Date de publication de la demande: **13.08.2025**
(21) Numéro de dépôt: 25155661.9
(22) Date de dépôt: 03.02.2025
(51) Int. Cl.: C09J 7/10, C09J 7/21, A61F 13/02

(54) **FILM ADHÉSIF RENFORCÉ, EN PARTICULIER POUR DES APPLICATIONS DANS LE DOMAINE MÉDICAL, ET PROCÉDÉ POUR FABRIQUER UN TEL FILM**

(30) Priorité: 09.02.2024 FR 2401287
(71) Demandeur: ADHEX TECHNOLOGIES, 21300 Chenôve (FR)
(72) Inventeur: WILLIAMS, Maud, 21000 DIJON (FR); MATRAY, Julie, 21110 FAUVERNEY (FR)
(74) Mandataire: Gevers & Orès

(57) **Abrégé**

- Le procédé de fabrication d'un film (1) adhésif renforcé comporte une unique étape d'enduction principale, mise en oeuvre par un dispositif d'enduction, consistant, lors d'un seul passage, à noyer complètement une trame (2) formée d'un non-tissé (3) dans une formulation adhésive (4) sensible à la pression de sorte que la formulation adhésive (4) recouvre complètement les deux faces (2A, 2B) de la trame (2), ce qui permet d'obtenir rapidement, à l'aide d'un procédé de fabrication simple, un film (1) adhésif renforcé présentant des propriétés avantageuses en particulier en termes d'adhérence, de tenue, de conformabilité et de respirabilité.

## Description

### Domaine technique

La présente invention concerne un film adhésif renforcé, en particulier pour des applications dans le domaine médical, ainsi qu'un procédé pour fabriquer un tel film.

### État de la technique

Le film adhésif renforcé peut être employé dans différents domaines, en particulier dans le domaine industriel et notamment dans le secteur du bâtiment, ainsi que dans le domaine médical, notamment pour l'adhésivation de tout type de champ opératoire.

Ce film adhésif, lorsqu'il est utilisé dans un bloc opératoire en milieu hospitalier, peut être appliqué par des chirurgiens lors d'opérations, pour un collage soit sur une table d'opération, soit directement sur un patient.

Il existe actuellement des adhésifs double-face, intégrant des formulations adhésives, qui sont utilisés pour de telles applications.

Seules, de telles formulations adhésives présentent plusieurs inconvénients. En particulier :
- il est nécessaire de les rendre plus consistantes, solides et résistantes, pour une utilisation en particulier dans le domaine médical, ce qui a tendance à réduire et limiter l'étendue de leurs propriétés ; et
- lorsqu'elles sont découpées en vue de leur utilisation, elles ont tendance à polluer les outils de découpe.

En revanche, les adhésifs double-face sont résistants lors des procédés de découpe et d'utilisation, mais présentent l'inconvénient d'être non perméables à l'eau, ce qui peut générer des décollements lorsqu'ils sont appliqués sur différents substrats.

Par ailleurs, la présence d'une trame centrale rigidifie l'ensemble, et diminue les performances adhésives du complexe.

Aussi, il existe un intérêt et un besoin de pouvoir disposer d'un film adhésif renforcé permettant de remédier à ces inconvénients.

### Exposé de l'invention

La présente invention a pour objet de proposer un film adhésif renforcé, susceptible d'être utilisé notamment dans les applications précitées et de répondre à ce besoin.

Selon l'invention, ledit film adhésif renforcé comprend une trame formée d'un non-tissé, qui est complètement noyée dans une formulation adhésive sensible à la pression de telle sorte que la formulation adhésive (englobe complètement la trame et) recouvre complètement les deux faces de la trame.

Ainsi, on obtient un film adhésif dont la tenue et la résistance sont renforcées, grâce à l'intégration de la trame (formée du non-tissé). Ceci permet également que les propriétés et performances initiales du film adhésif ainsi renforcé ne soient pas détériorées au cours du temps, notamment lorsqu'il est soumis à diverses étapes de transformation comme cela est généralement le cas.

L'ajout de la trame dans la formulation adhésive permet, en plus de renforcer la tenue du film adhésif, d'améliorer sa stabilité au fluage lors du stockage du produit fini comprenant ce film adhésif ainsi que de faciliter la découpe du film (et ceci sans polluer les outils de découpe).

De plus, le caractère non occlusif dudit film adhésif est également un avantage concernant la tenue à l'humidité sur différentes surfaces.

Grâce à ses propriétés avantageuses précisées ci-dessous, ce film adhésif renforcé est particulièrement bien adapté à une utilisation dans le domaine médical, notamment dans un bloc opératoire pour être appliqué par des chirurgiens lors d'opérations, par collage soit sur une table d'opération, soit directement sur un patient. Ce film adhésif renforcé pourra remplacer avantageusement les adhésifs double-face usuels, en permettant de remédier à leurs inconvénients précisés ci-dessus.

Dans un mode de réalisation préféré, le film adhésif renforcé présente, au moins certaines des caractéristiques suivantes :
- un taux de transmission de vapeur d'eau, mesuré selon la méthode de mesure de la norme NF EN 13726-2, supérieur à 400 g/m²/24h, illustrant ses propriétés de respirabilité ;
- un pouvoir adhésif 1 minute à 90° sur verre supérieur à 300 N/m ; et
- une conformabilité augmentée (par rapport notamment à des adhésifs double-face), comme précisé ci-dessous.

De plus, le non-tissé formant la trame présentant un grammage, mesuré selon la méthode de mesure de la norme FTM12, compris entre 3 et 8 g/m².

Ces caractéristiques permettent au film d'obtenir les propriétés précitées.

Dans un premier mode de réalisation, la formulation adhésive est un polymère acrylique réticulable sous rayons ultraviolets, avec pour monomères de base du butyle acrylate et de l'acide acrylique.

De préférence, ce polymère acrylique est formulé avec une résine tackifiante de nature hydrocarbonée.

En outre, dans un deuxième mode de réalisation, la formulation adhésive est un polymère acrylique en phase solvant, avec pour monomère de base de l'acrylate de 2-éthylhexyle.

Par ailleurs, dans un troisième mode de réalisation, la formulation adhésive est un élastomère de type styrène-isoprène-styrène ou de type styrène-butadiène-styrène.

De préférence, l'élastomère est formulé avec au moins l'un des types de composants suivants : résine, plastifiant, anti-oxydant.

Par ailleurs, avantageusement, le non-tissé formant la trame est en polyester, et présente, au moins certaines des caractéristiques suivantes :
- une épaisseur, mesurée selon la méthode de mesure de la norme NF EN ISO 534, comprise entre 0.03 et 0.07 mm ;
- une résistance à la rupture, mesurée selon la méthode de mesure de la norme NF EN 29073-3, comprise entre 20 et 30 N/5cm ;
- un allongement à la rupture, mesuré selon la méthode de mesure de la norme NF EN 29073-3, qui est compris entre 4 et 8 % ;
- un taux de transmission de vapeur d'eau, mesuré selon la méthode de mesure de la norme NF EN 13726-2, qui est supérieur à 10 000 g/m²/24h.

De préférence, les fibres du non-tissé formant la trame sont orientées sensiblement dans une seule et même direction de manière notamment à augmenter la résistance du film adhésif.

En outre, dans un mode de réalisation préféré, le film adhésif renforcé comprend un film protecteur qui est siliconé sur ses deux faces et qui supporte sur l'une de ses faces siliconées l'ensemble formé de la formulation adhésive dans laquelle est noyée la trame.

La présente invention concerne également un procédé de fabrication d'un film adhésif renforcé.

Selon l'invention, ledit procédé comprend une unique étape d'enduction principale, mise en oeuvre par un dispositif d'enduction, consistant, lors d'un seul passage, à noyer complètement une trame formée d'un non-tissé dans une formulation adhésive sensible à la pression de sorte que la formulation adhésive (englobe complètement la trame et) recouvre complètement les deux faces de la trame. De plus, ledit film adhésif renforcé présente un taux de transmission de vapeur d'eau, mesuré selon la norme NF EN 13726-2, supérieur à 400 g/m²/24h, un pouvoir adhésif 1 min à 90° sur verre supérieur à 300 N/m, et une conformabilité augmentée par rapport à des adhésifs double-face, et le non-tissé formant la trame présente un grammage, mesuré selon FTM12, compris entre 3 et 8 g/m².

Ainsi, grâce à l'invention, le film adhésif renforcé (qui présente les caractéristiques avantageuses précitées) est obtenu lors d'un seul passage au cours duquel le non-tissé est complètement noyé dans la formulation adhésive. Ceci permet d'obtenir un procédé de fabrication particulièrement simple et une fabrication rapide, comparé à des procédés usuels qui comprennent au moins deux passages (à savoir un premier passage pour déposer un adhésif sur une première face d'un support et un second passage pour déposer un adhésif sur la seconde face du support).

Avantageusement, le non-tissé de la trame présente des caractéristiques appropriées permettant à la formulation adhésive de le traverser et de se trouver des deux côtés du non-tissé. Ces caractéristiques sont, de préférence, adaptées aux caractéristiques de la formulation adhésive.

Le procédé comprend également une étape d'enduction auxiliaire qui consiste à déposer la formulation adhésive sur un film protecteur, et qui :
- dans un premier mode de réalisation, est mise en oeuvre antérieurement à l'étape d'enduction principale ; et
- dans un second mode de réalisation, est mise en oeuvre en même temps que l'étape d'enduction principale.

De façon avantageuse, la formulation adhésive utilisée est soit thermofusible, soit en phase solvant.

### Brève description des figures

D'autres avantages et caractéristiques ressortiront mieux de la description qui va suivre de plusieurs modes de réalisation, donnés à titre d'exemples non limitatifs, d'un film adhésif renforcé conforme à l'invention et de son procédé de fabrication, en se référant notamment aux figures annexées. Sur ces figures, des références identiques désignent des éléments semblables.
La figure 1 est une vue schématique partielle, en coupe, d'un film adhésif renforcé, conforme à un mode de réalisation de l'invention.
La figure 2 est une vue schématique d'un premier mode de réalisation d'un dispositif de fabrication d'un film adhésif renforcé.
La figure 3 est une vue schématique d'un deuxième mode de réalisation d'un dispositif de fabrication d'un film adhésif renforcé.
La figure 4 est une vue schématique d'une variante du deuxième mode de réalisation de la figure 3.

### Description détaillée

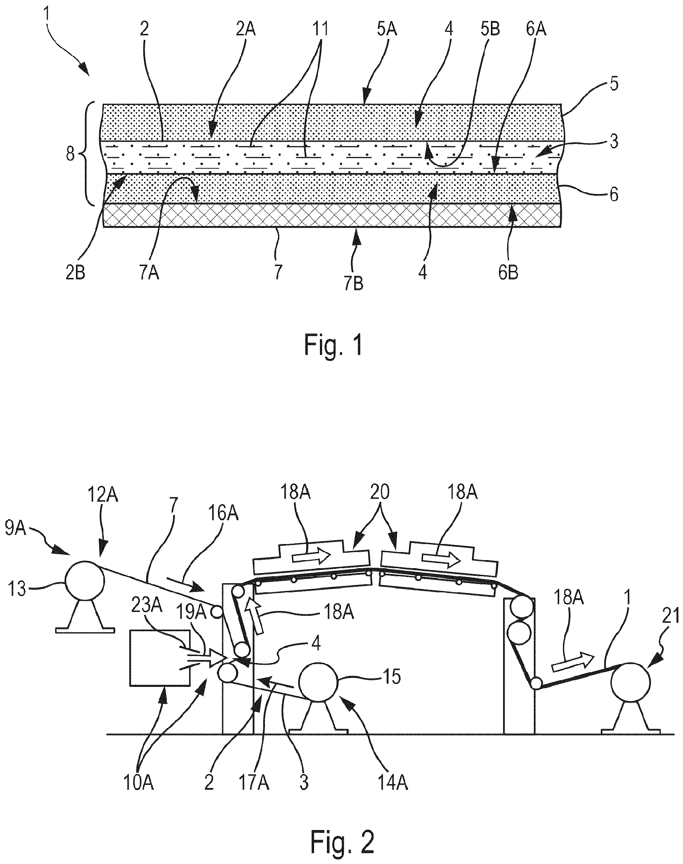

Le film 1 permettant d'illustrer l'invention et représenté partiellement selon un mode de réalisation particulier sur la figure 1 est un film adhésif renforcé, comme précisé ci-dessous.

Comme représenté sur la figure 1, le film 1 comprend une trame 2 formée d'un non-tissé 3.

Cette trame 2 est complètement noyée dans une formulation adhésive 4 sensible à la pression de telle sorte que la formulation adhésive 4 englobe complètement la trame 2 et recouvre complètement les deux faces 2A et 2B de la trame 2. Ainsi, deux couches adhésives 5 et 6 sont formées de part et d'autre de la trame 2 en non-tissé 3. La couche adhésive 5 est formée sur la face 2A de la trame 2 et la couche adhésive 6 est formée sur la face 2B de la trame 2.

Comme précisé ci-dessous, la formulation adhésive 4 utilisée pour fabriquer le film 1 est soit thermofusible (« hotmelt »), soit en phase solvant. La formulation adhésive 4 est représentée très schématiquement par de petits points noirs sur les figures 1, 3 et 4.

Le film 1 comprend également un film protecteur 7, sur l'une des faces de l'ensemble 8 formé de la formulation adhésive 4 dans laquelle est noyée la trame 2.

Dans l'exemple de la figure 1, le film protecteur 7 est en contact par une face 7A d'une face 6B de la couche adhésive 6 (qui est opposée à la face 6A en contact avec la face 2B de la trame 2). La couche adhésive 5 comprend deux faces 5A et 5B dont la face 5B est en contact de la face 2A de la trame 2.

De préférence, le film protecteur 7 est un papier glassine, siliconé sur ses deux faces 7A et 7B. Ceci qui permet d'enrouler le film 1 sur lui-même, la face 5A du film 1 venant au contact de la face 7B (siliconée) du film protecteur 7 lors du bobinage.

Dans un mode de réalisation préféré, le film 1 adhésif renforcé présente au moins certaines des caractéristiques suivantes :
- un pouvoir adhésif 1 min à 90° sur verre, supérieur à 300 N/m en face vue et en face cachée, illustrant ses propriétés d'adhérence ;
- une force de décomplexage en T entre le film protecteur 7 et la couche adhésive 6 comprise entre 10 et 60 cN/5cm ;
- un taux de transmission de vapeur d'eau (MVTR pour « Moisture Vapour Transmission Rate » en anglais), à savoir le débit de perméation de l'eau à travers une membrane, mesuré (selon la méthode de mesure de la norme NF EN 13726-2) en masse par unité de surface par unité de temps, en l'occurrence en g/m²/24h, qui est supérieur à 400 g/m²/24h (en face cachée et en face vue), illustrant ses propriétés de respirabilité ;
- une force de décomplexage en T sur champ opératoire (film) après 24h, supérieure à 500 cN/5cm ;
- une force de décomplexage en T sur champ opératoire (non-tissé) après 24h, supérieure à 500 cN/5cm.

Dans le cadre de la présente invention, les plages de valeurs présentées sous forme de « entre x et y » incluent les bornes x et y, les entiers compris entre ces bornes, ainsi que tous les autres nombres réels compris entre ces bornes.

Ainsi, on obtient un film 1 adhésif dont la tenue et la résistance sont renforcées, grâce à l'intégration de la trame 2 (formée du non-tissé 3). De plus, les propriétés et les performances initiales du film 1 adhésif ainsi renforcé ne sont pas détériorées au cours du temps, notamment lorsqu'il est soumis à diverses étapes de transformation comme cela est généralement le cas. L'ajout de la trame 2 dans la formulation adhésive 4 permet également d'améliorer sa stabilité au fluage lors du stockage du produit fini comprenant ce film 1, et de faciliter la découpe du film 1 (et ceci sans polluer les outils de découpe utilisés à cet effet).

Dans le cadre de la présente invention, la formulation adhésive 4 utilisée pour fabriquer le film 1 peut être réalisée de différentes manières.

Dans un premier mode de réalisation, la formulation adhésive 4 correspond à un polymère acrylique réticulable sous rayons ultraviolets (UV), avec pour monomères de base du butyle acrylate (BA) et de l'acide acrylique (AA).

De préférence, ce polymère acrylique est formulé avec une résine tackifiante de nature hydrocarbonée, afin d'apporter une adhésivité particulière au film 1 (si nécessaire ou si utile).

En outre, dans un deuxième mode de réalisation, la formulation adhésive 4 correspond à un polymère acrylique en phase solvant, non réticulable sous rayons ultraviolets, à base de monomère EHA (acrylate de 2-éthylhexyle).

Par ailleurs, dans un troisième mode de réalisation, la formulation adhésive 4 correspond à un élastomère de type styrène-isoprène-styrène (SIS) ou de type styrène-butadiène-styrène (SBS).

De préférence, cet élastomère est formulé avec au moins l'un des types de composants suivants : résine, plastifiant, anti-oxydant, afin d'apporter les propriétés souhaitées au film 1.

On notera que le choix concernant la nature chimique (acrylique ou élastomère) de la formulation adhésive 4 peut dépendre du ou des substrats de l'application envisagée et du temps de pose de la formulation adhésive 4.

A titre d'illustration, le tableau ci-dessous permet de mettre en évidence les caractéristiques principales de la formulation adhésive 4 pour quatre exemples différents nommés FA1 à FA4, qui sont susceptibles d'être utilisés pour réaliser le film 1.

| Référence commerciale | FA1 : HM-R2292 | FA2 : HM-R2519A | FA3 : AS-333 | FA4 : AS-1959 |
|---|---|---|---|---|
| Technologie | thermofusible | thermofusible | phase solvant | phase solvant |
| Composition des monomères constituant l'acrylique (%) | 94% d'acrylate de butyle | 89.3% d'acrylate de butyle | acrylate de 2-ethylhexyle | acrylate de 2-ethylhexyle |
| | 4% d'acide acrylique | 3.8% d'acide acrylique | acétate de vinyle | acétate de vinyle |
| | 2% d'acrylate d'éthyle | 1.9% d'acrylate d'éthyle | acide acrylique | acide acrylique |
| | | 5% résine tackifiante aromatique | | |
| Viscosité Brookfield (mPa.s) | 130°C : | 130°C : | 25°C : | 25°C : |
| | vitesse 1 : 75 000 | vitesse 1 : 54 750 | 3200 - 5800 | 4000-10000 |
| | vitesse 25 : 71 000 | vitesse 2.5 : 47 100 | | |
| | vitesse 5 : 66 000 | vitesse 5 : 43 550 | | |

Ce tableau présente pour les quatre exemples FA1 à FA4 considérés de formulation adhésive 4 :
- leur référence commerciale ;
- la technologie (thermofusible ou en phase solvant) ;
- la composition des monomères constituant l'acrylique, en pourcentage (%) ; et
- la viscosité « Brookfield » en mPa.s (mesurée selon la méthode de mesure de la norme NF EN ISO 2555).

Par ailleurs, dans un mode de réalisation préféré, le non-tissé 3 formant la trame 2 est très aéré, en particulier pour permettre le passage de la formulation adhésive 4. De préférence, le non-tissé 3 est composé de fibres en polyester.

Dans un mode de réalisation préféré, les fibres 11 du non-tissé 3 sont de type monodirectionnel et sont donc orientées selon une seule et même direction. Ceci permet notamment d'augmenter la résistance du film 1.

En comparaison avec des non-tissés de technologie « spunbond », des épaisseurs plus fines peuvent ainsi être obtenues pour le non-tissé 3 pour un grammage identique.

Dans un mode de réalisation préféré, le non-tissé 3 présente au moins certaines des caractéristiques suivantes :
- un grammage compris entre 3 et 8 g/m² (mesuré selon la méthode de mesure de la norme FTM12 (« FINAT Test Method »)) ;
- une épaisseur comprise entre 0.03 et 0.07 mm (mesurée selon la méthode de mesure de la norme NF EN ISO 534) ;
- une résistance à la rupture comprise entre 20 et 30 N/5cm (mesurée selon la méthode de mesure de la norme NF EN 29073-3) en direction de la machine (MD pour « machine direction » en anglais) ;
- un allongement à la rupture qui est compris entre 4 et 8 % (mesuré selon la méthode de mesure de la norme NF EN 29073-3) ;
- un taux de transmission de vapeur d'eau (MVTR pour « Moisture Vapour Transmission Rate » en anglais), à savoir le débit de perméation de l'eau à travers une membrane, mesuré (selon la méthode de mesure de la norme NF EN 13726-2) en masse par unité de surface par unité de temps, en l'occurrence en g/m²/24h, qui est supérieur à 10 000 g/m²/24h.

Le film 1, tel que décrit ci-dessus, peut être obtenu à l'aide d'un procédé de fabrication précisé ci-dessous. Ce procédé de fabrication, de préférence en grande laize, comprend, notamment, une étape d'enduction principale en un seul passage.

Cette (unique) étape d'enduction principale qui est mise en oeuvre par un dispositif d'enduction, consiste, lors d'un seul passage, à noyer complètement une trame 2 formée d'un non-tissé 3 dans une formulation adhésive 4 sensible à la pression de sorte que la formulation adhésive 4 englobe complètement la trame 2 et recouvre complètement les deux faces 2A et 2B de la trame 2 (figure 1).

Ainsi, le film (adhésif renforcé) 1 qui présente les caractéristiques avantageuses précitées, est obtenu lors d'un seul passage (au niveau du dispositif d'enduction) au terme duquel le non-tissé 3 est complètement noyé dans la formulation adhésive 4.

Pour ce faire, le non-tissé 3 comprend des caractéristiques appropriées et est notamment très aéré pour permettre à la formulation adhésive 4 de le traverser et d'être présente des deux côtés du non-tissé 3, par exemple lorsque le non-tissé 3 passe sous une buse d'enduction (amenant la formulation adhésive 4) du dispositif d'enduction. Ces caractéristiques du non-tissé 3 de la trame 2 (telles que le fait d'être aéré) peuvent être adaptées aux caractéristiques (par exemple la viscosité) de la formulation adhésive 4 pour permettre à la formulation adhésive 4 d'être présente des deux côtés de la trame 2 et de l'envelopper complètement.

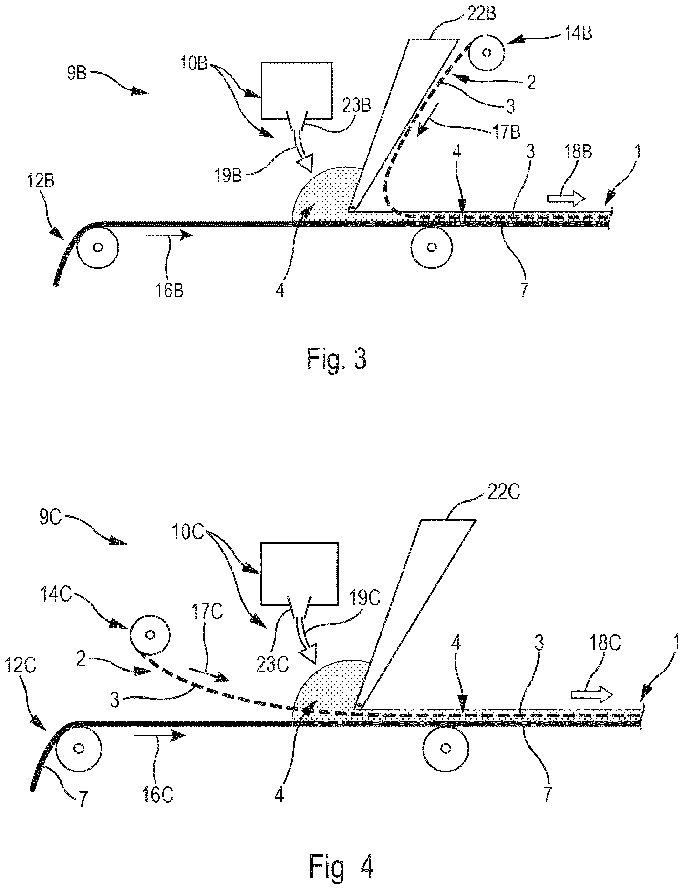

Le procédé de fabrication comprend également une étape d'enduction dite auxiliaire, consistant à déposer la formulation adhésive 4 sur le film protecteur 7. La trame 2 peut être noyée dans la formulation adhésive 4 en même temps que cette dernière est déposée sur le film protecteur 7 ou après que la formulation adhésive 4 a été déposée sur le film protecteur 7. Ainsi, dans une première mise en oeuvre correspondant aux modes de réalisation des figures 2 et 4 décrits ci-dessous, l'étape d'enduction auxiliaire est mise en oeuvre en même temps que l'étape d'enduction principale, et dans une seconde mise en oeuvre correspondant au mode de réalisation de la figure 3, l' étape d'enduction auxiliaire (dépôt de la formulation adhésive 4 sur le film protecteur 7) est mise en oeuvre antérieurement à l'étape d'enduction principale (intégration de la trame 2 dans la formulation adhésive 4).

Dans le cadre de la présente invention, le procédé de fabrication, et notamment son étape d'enduction principale, peuvent être mis en oeuvre à partir de différents dispositifs d'enduction tels que, par exemple, ceux décrits ci-dessous, à titre d'illustration, en référence aux figures 2 à 4.

Dans un premier mode de réalisation représenté sur la figure 2, le procédé de fabrication utilise un dispositif de fabrication 9A qui comprend un dispositif d'enduction 10A. Le dispositif de fabrication 9A est configuré pour mettre en oeuvre un procédé « hotmelt » (thermofusible) en grande laize (1500 mm).

Dans ce premier mode de réalisation, le dispositif de fabrication 9A comprend, de plus, un système d'alimentation 12A en film protecteur 7, pourvu notamment d'une bobine 13 de film protecteur 7, et un système d'alimentation 14A en non-tissé 3, pourvu notamment d'une bobine 15 de non-tissé 3.

Le non-tissé 3 et le film protecteur 7 sont amenés, par ces systèmes d'alimentation 12A et 14A, dans les sens illustrés respectivement par des flèches 16A et 17A, au dispositif d'enduction 10A. Le dispositif d'enduction 10A est pourvu au moins d'une buse d'enduction 23A et est alimenté, de façon usuelle, en formulation adhésive 4. Le film protecteur 7 qui sert de support à la formulation adhésive 4 thermofusible dans laquelle est noyée la trame 2 en non-tissé 3 (également amené à ce niveau), passe sous la ou les buses d'enduction 16A (pour recevoir la formulation adhésive 4 comme illustré par une flèche 19A) de sorte que, en lien avec les caractéristiques du non-tissé 3, la formulation adhésive 4 thermofusible se répartisse des deux côtés de la trame 2 en non-tissé 3.

L'ensemble (formé de ce non-tissé 3 qui est noyé dans la formulation adhésive 4 déposée sur le film protecteur 7) est amené, dans le sens illustré par des flèches 18A, à travers un système de séchage 20 (ou tunnel de séchage ou four(s)), puis est bobiné au niveau d'un poste de bobinage 21, en aval du système de séchage 20. A ce poste de bobinage 21, le film 1 adhésif renforcé qui vient d'être fabriqué est enroulé sur une bobine.

Dans une variante de réalisation (non représentée), le système de séchage 20 est remplacé, de façon usuelle, par des lampes UV, lors notamment de l'utilisation d'acryliques thermofusibles (« hotmelt ») réticulables sous rayons ultraviolets (UV) pour la formulation adhésive 4.

Dans un deuxième mode de réalisation représenté sur la figure 3, le procédé de fabrication utilise un dispositif de fabrication 9B comprenant un dispositif d'enduction 10B configuré pour mettre en oeuvre une fabrication en phase solvant, en grande laize (1500 mm).

Dans ce deuxième mode de réalisation, le dispositif de fabrication 9B comprend, de plus, un système d'alimentation 12B en film protecteur 7 et un système d'alimentation 14B en non-tissé 3.

Le non-tissé 3 et le film protecteur 7 sont amenés, par ces systèmes d'alimentation 12B et 14B, dans les sens illustrés respectivement par des flèches 16B et 17B, au dispositif d'enduction 10B. Le dispositif d'enduction 10B est pourvu au moins d'un système d'alimentation 23B qui alimente, de façon usuelle, la formulation adhésive 4.

Le dispositif d'enduction 10B est configuré pour déposer la formulation adhésive 4 en phase solvant sur le film protecteur 7, comme illustré par une flèche 19B, en amont d'une racle 22B du dispositif d'enduction 10B, dans le sens de déplacement (illustré par une flèche 18B) du film 1.

Le non-tissé 3 (fourni par le système d'alimentation 14B en non-tissé) est, quant à lui, intégré en aval de la racle 22B, dans la formulation adhésive 4 préalablement déposée sur le film protecteur 7.

En aval de cette intégration, l'ensemble (formé de ce non-tissé 3 qui est noyé dans la formulation adhésive 4 déposée sur le film protecteur 7) est soumis à des traitements usuels, notamment de séchage et de bobinage, à l'aide de dispositifs usuels (non représentés).

Par ailleurs, dans un troisième mode de réalisation représenté sur la figure 4 qui est une variante du deuxième mode de réalisation de la figure 3, le procédé de fabrication utilise un dispositif de fabrication 9C comprenant un dispositif d'enduction 10C. A la différence du dispositif d'enduction 10B du deuxième mode de réalisation, le dispositif d'enduction 10C est configuré pour que le non-tissé 3 soit intégré dans la formulation adhésive 4 déposée sur le film protecteur 7, en amont d'une racle 22C dans le sens de déplacement (illustré par une flèche 18C) du film 1.

Le système d'alimentation 14C est donc agencé en amont de la racle 22C. L'essentiel des autres éléments du dispositif de fabrication 9C sont identiques ou similaires à ceux du dispositif de fabrication 9B. En particulier, le non-tissé 3 et le film protecteur 7 sont amenés, par des systèmes d'alimentation 12C et 14C, dans le sens illustré respectivement par des flèches 16C et 17C, au dispositif d'enduction 10C pourvu au moins d'un système d'alimentation 23C fournissant la formulation adhésive 4 (flèche 19C).

Quel que soit le dispositif d'enduction 10A, 10B et 10C et le dispositif de fabrication 9A, 9B et 9C utilisés, le procédé de fabrication mis en oeuvre utilise un non-tissé 3, une formulation adhésive 4 et un film protecteur 7, tels que ceux décrits ci-dessus.

En particulier, il prend en compte les caractéristiques (telles que l'épaisseur, le grammage, ...) précisées ci-dessus du non-tissé 3, les caractéristiques précisées ci-dessus de la formulation adhésive 4 et les caractéristiques précisées ci-dessus du film protecteur 7.

Les dispositifs de fabrication 9A, 9B et 9C décrits ci-dessus permettent de réaliser une enduction, en grande laize, d'une formulation adhésive 4 sur un non-tissé 3 en un seul passage. On dispose ainsi d'un procédé de fabrication simple et rapide, comparé à des procédés de fabrication usuels qui comprennent au moins deux passages (à savoir un premier passage pour déposer un adhésif sur une première face d'un support et un second passage pour déposer un adhésif sur la seconde face du support).

De plus, le film 1 adhésif renforcé, ainsi obtenu, présente les caractéristiques particulièrement avantageuses précisées ci-dessus.

Le tableau ci-après présente un comparatif entre un exemple F1 de film 1 (obtenu par un procédé conforme à un mode de réalisation particulier de l'invention) et un adhésif double-face usuel connu (P7592) et permet de mettre en évidence les caractéristiques avantageuses du film 1.

| | F1 | | P7592 |
|---|---|---|---|
| Construction | Adhésif | HM-R 2519A, 60 g/m² | face vue : HMR-2292, 60g/m² |
| | | | face cachée : HMR-2292, 40g/m² |
| | Trame | non-tissé monodirectionnel | aucune |
| | Support | aucun | film PET, 17g/m² |
| | Film protecteur | papier siliconé 2F différenciées | papier siliconé 2F non différenciées |
| Grammage g/m² | | 60 | 100 |
| PA 90° sur verre 1 min - Face vue - cN/cm | | 467 | 750 |
| PA 90° sur verre 1 min - Face cachée - cN/cm | | 565 | 825 |
| MVTR contact vapeur (g/m²/24h) | | 870 | 45 |

Ce tableau présente pour les deux produits considérés :
- les caractéristiques de l'adhésif (ou formulation adhésive) utilisé ;
- la présence ou non d'une trame et d'un support ;
- les caractéristiques du film protecteur utilisé ;
- le grammage (mesuré selon la méthode de mesure de la norme FTM12) en g/m² ;
- le pouvoir adhésif PA 90° sur verre 1 minute, respectivement pour la face vue et la face cachée, en cN/cm ; et
- le taux de transmission de vapeur d'eau (MTVR), mesuré selon la norme NF EN 13726-2, en g/m²/24h.

Ce tableau permet notamment de mettre en évidence les caractéristiques avantageuses suivantes du film 1 :
- un taux de transmission de vapeur d'eau compris supérieur à 400 g/m²/24h, illustrant ces propriétés de respirabilité ;
- un pouvoir adhésif supérieur à 300 N/m ; et
- une conformabilité supérieure à celle des adhésifs double-face.

La conformabilité améliorée du film 1 (c'est-à-dire supérieure à celle des adhésifs double-face) peut être mise en évidence par un test mécanique d'hystérésis et un test illustrant la souplesse.

Concernant le test d'hystérésis, des mesures de déformation à 20% ont été réalisées, à la fois, sur le film 1 et sur l'adhésif double-face usuel précité (P7592), pour des éprouvettes de 15 mm de laize et 10 cm de longueur.

Le tableau, ci-après, présente un comparatif entre l'exemple F1 de film 1 (obtenu par un procédé conforme à un mode de réalisation particulier de l'invention) et l'adhésif double-face usuel connu (P7592), montrant les valeurs de différents paramètres, et il permet de mettre en évidence les caractéristiques avantageuses du film 1 concernant la conformabilité.

| | Force d'hystérésis (N/cm) | Rémanence (%) | Pente au départ (N/cm/%) |
|---|---|---|---|
| F1 | 6,4 | 14,9 | 0,6 |
| P7592 | 13,5 | 15,4 | 4,5 |

Ce tableau présente pour les deux produits considérés :
- la force d'hystérésis, en N/cm ;
- la rémanence, en % ; et
- la pente au départ de la courbe d'hystérésis, en N/cm/%.

On constate que l'écart entre les mesures en termes de rémanence est faible, mais l'adhésif double-face est, toutefois, plus résistant à la déformation que le film 1.

Quant aux pentes au démarrage des courbes d'hystérésis (et donc les modules d'Young), elles montrent une différence de comportement entre les deux produits analysés. L'adhésif double-face est bien plus rigide que le film 1.

En outre, concernant le test de souplesse à la boucle, des éprouvettes de 20 cm de longueur et de 2 cm de laize, ont été utilisées. Pour chacun des deux produits, l'éprouvette correspondante a été maintenue par ses extrémités libres, amenés en contact et disposés verticalement vers le haut, de manière à former une boucle.

Dans ce test, on observe une forme de boucle différente pour les deux produits, avec une largeur de la boucle maximale :
- de 2,5 cm pour le film 1 ; et
- de 4 cm pour l'adhésif double-face usuel (P7592).

Ce test permet de démontrer la souplesse du film 1 (renforcé par la trame 2) par rapport à l'adhésif double-face (en polyester).

La conformabilité améliorée du film 1, par rapport à un adhésif double-face usuel, a ainsi été mise en évidence par ces deux tests, le test mécanique d'hystérésis et le test relatif à la souplesse.

Le film 1, tel que décrit ci-dessus, qui présente des propriétés adhésives et cohésives avantageuses, est particulièrement bien adapté à une utilisation dans un bloc opératoire, et peut notamment être appliqué par les chirurgiens lors d'opérations en milieu hospitalier, pour un collage soit sur une table d'opération, soit directement sur un patient.

Le film 1, tel que décrit ci-dessus, peut être utilisé dans de nombreuses autres applications, en particulier (mais non exclusivement) dans le secteur médical. Plus généralement, le film 1 peut être utilisé dans toutes les applications dans lesquelles ses caractéristiques avantageuses (et notamment ses propriétés d'adhésif renforcé) sont recherchées.

Il peut en particulier être utilisé dans le domaine industriel, et notamment dans le secteur du bâtiment, dans lequel on peut notamment tirer profit de ses propriétés de respirabilité.

Il est bien évident que les exemples présentés ci-dessus ne sont que des illustrations particulières, en aucun cas limitatives quant aux domaines d'application de la présente invention. De plus, des caractéristiques de certains de ces exemples différents peuvent être combinées entre elles si cela est approprié, sans sortir du cadre de la présente invention.

## Revendications

1. Film adhésif renforcé, en particulier pour le domaine médical, ledit film adhésif renforcé (1) comprenant une trame (2) formée d'un non-tissé (3), qui est complètement noyée dans une formulation adhésive (4) sensible à la pression de telle sorte que la formulation adhésive (4) recouvre complètement les deux faces (2A, 2B) de la trame (2), ledit film adhésif renforcé (1) présentant un taux de transmission de vapeur d'eau, mesuré selon la norme NF EN 13726-2, supérieur à 400 g/m²/24h, un pouvoir adhésif 1 min à 90° sur verre supérieur à 300 N/m, et une conformabilité augmentée par rapport à des adhésifs double-face, le non-tissé (3) formant la trame (2) présentant un grammage, mesuré selon FTM12, compris entre 3 et 8 g/m².

2. Film selon la revendication 1,
**caractérisé en ce que** la formulation adhésive (4) est un polymère acrylique réticulable sous rayons ultraviolets, avec pour monomères de base du butyle acrylate et de l'acide acrylique.

3. Film selon la revendication 2,
**caractérisé en ce que** le polymère acrylique est formulé avec une résine tackifiante de nature hydrocarbonée.

4. Film selon la revendication 1,
**caractérisé en ce que** la formulation adhésive (4) est un polymère acrylique en phase solvant, avec pour monomère de base de l'acrylate de 2-éthylhexyle.

5. Film selon la revendication 1,
**caractérisé en ce que** la formulation adhésive (4) est un élastomère de type styrène-isoprène-styrène ou de type styrène-butadiène-styrène.

6. Film selon la revendication 5,
**caractérisé en ce que** l'élastomère est formulé avec au moins l'un des types de composants suivants : résine, plastifiant, anti-oxydant.

7. Film selon l'une quelconque des revendications précédentes,
**caractérisé en ce que** le non-tissé (3) formant la trame (2) est en polyester, et présente au moins certaines des caractéristiques suivantes :
- une épaisseur, mesurée selon la norme NF EN ISO 534, comprise entre 0.03 et 0.07 mm, ;
- une résistance à la rupture, mesurée selon la norme NF EN 29073-3, comprise entre 20 et 30 N/5cm ;
- un allongement à la rupture, mesuré selon la norme NF EN 29073-3, qui est compris entre 4 et 8 % ;
- un taux de transmission de vapeur d'eau, mesuré selon la norme NF EN 13726-2, qui est supérieur à 10 000 g/m²/24h.

8. Film selon l'une quelconque des revendications précédentes,
**caractérisé en ce que** les fibres (11) du non-tissé (3) formant la trame (2) sont orientées sensiblement dans une seule et même direction.

9. Film selon l'une quelconque des revendications précédentes,
**caractérisé en ce qu'**il comprend un film protecteur (7) qui est siliconé sur ses deux faces (7A, 7B) et qui supporte, sur l'une (7A) de ses faces (7A, 7B) siliconées, l'ensemble (8) formé de la formulation adhésive (4) dans laquelle est noyée la trame (2).

10. Procédé de fabrication d'un film adhésif renforcé,
**caractérisé en ce qu'**il comprend une unique étape d'enduction principale, mise en oeuvre par un dispositif d'enduction (10A, 10B, 10C), consistant, lors d'un seul passage, à noyer complètement une trame (2) formée d'un non-tissé (3) dans une formulation adhésive (4) sensible à la pression de sorte que la formulation adhésive (4) recouvre complètement les deux faces (2A, 2B) de la trame (2), ledit film adhésif renforcé (1) présentant un taux de transmission de vapeur d'eau, mesuré selon la norme NF EN 13726-2, supérieur à 400 g/m²/24h, un pouvoir adhésif 1 min à 90° sur verre supérieur à 300 N/m, et une conformabilité augmentée par rapport à des adhésifs double-face, le non-tissé (3) formant la trame (2) présentant un grammage, mesuré selon FTM12, compris entre 3 et 8 g/m².

11. Procédé selon la revendication 10,
**caractérisé en ce qu'**il comprend une étape d'enduction auxiliaire consistant à déposer la formulation adhésive (4) sur un film protecteur (7), qui est mise en oeuvre antérieurement à l'étape d'enduction principale.

12. Procédé selon la revendication 10,
**caractérisé en ce qu'**il comprend une étape d'enduction auxiliaire consistant à déposer la formulation adhésive (4) sur un film protecteur (7), qui est mise en oeuvre en même temps que l'étape d'enduction principale.

13. Procédé selon l'une des revendications 10 à 12,
**caractérisé en ce que** la formulation adhésive (4) utilisée est soit thermofusible, soit en phase solvant.
